Europäisches Patentamt

European Patent Office

Office européen des brevets

(19)

(11) Publication number: **0 040 087**
**B1**

# EUROPEAN PATENT SPECIFICATION

(12)

(45) Date of publication of patent specification: 05.09.84

(21) Application number: 81302086.4

(22) Date of filing: 11.05.81

(51) Int. Cl.³: **A 61 L 15/00, D 06 M 15/38 //**
**C08F2/54, C08F20/04**

(54) Absorbent composite.

(30) Priority: 12.05.80 US 149215

(43) Date of publication of application:
18.11.81 Bulletin 81/46

(45) Publication of the grant of the patent:
05.09.84 Bulletin 84/36

(84) Designated Contracting States:
DE FR GB NL

(56) References cited:
DE-A-2 737 994
FR-A-2 140 323
FR-A-2 368 994

(73) Proprietor: Johnson & Johnson
501 George Street
New Brunswick, New Jersey 08903 (US)

(72) Inventor: Ericksen, Paul Herbert
29 Fairview Avenue
East Brunswick New Jersey 08816 (US)
Inventor: Nguyen, Hien Vu
5 Amy Drive
East Windsor New Jersey 08520 (US)
Inventor: Oczkowski, Boguslaw
3, Easton Avenue
Spotswood New Jersey 08884 (US)
Inventor: Olejnik, Thomas Andrew
5807 Fox Run Drive
Plainsboro New Jersey (US)

(74) Representative: Jones, Alan John et al
CARPMAELS & RANSFORD 43 Bloomsbury
Square
London, WC1A 2RA (GB)

Courier Press, Leamington Spa, England.

## Description

### Background of the Invention

Absorbent composites are widely employed as diapers, wound dressings, sanitary products, bandages or incontinence pads. Considerable research has been done in the last decade or so in attempting to incorporate the so-called "superabsorbent" polymers in absorbent products. Superabsorbent polymers are generally considered to be hydrophilic polymers that swell when they contact water, but which are not soluble in water. Such polymers usually have the theoretical capacity to absorb many times their own weight in distilled or deionized water.

Despite the obvious advantage of employing superabsorbent polymers in absorbent products, commercial absorbent materials containing superabsorbent polymers have been slow in coming to the market place. It has been difficult to utilize the potential of superabsorbent polymers in composite structures for a variety of reasons. First, if the superabsorbent polymer is present in a fairly large mass, when aqueous fluid contacts the polymer a gel is formed on the surface which slows down or even prevents the passage of additional fluid through the gel to reach the remainder of the superabsorbent polymer. Attempts to avoid this problem by employing finely divided granules of superabsorbent polymers have met with limited success because it is difficult to affix the granules in place. In some cases, it has been attempted to employ superabsorbent polymers in the form of films in absorbent products. However, the film acts as an effective barrier to the passage of fluid therethrough, whichis a limitation on the flexibility of such materials in that the article is usually designed for use in such a way that fluid is not expected to pass through the film.

In accordance with the present invention, a method has been discovered which can be employed to produce an absorbent composite containing a water-swellable polymer. The method enables the production of a water-swellable polymer that is firmly affixed in place in a fibrous substrate, and which has flexbile design capabilities in that the water-swellable polymer can be incorporated in the said substrate in many different forms. Thus, the polymer can be incorporated in the substrate in a configuration having a high surface area to mass ratio, to thereby increase the take-up rate of the aqueous fluid to be absorbed, and also to more nearly utilize the theoretical absorption limit of the polymer. The polymer can also contribute to the mechanical strength of the substrate, and can therefore replace some or all of the conventional binder in a nonwoven fabric and can enhance the stability of a fluffy batt of fibers.

### Brief Summary of the Invention

The invention provides an absorbent composite which is produced by a process which comprises:

(a) applying an aqueous solution comprising an ammonium or alkali metal salt of acrylic or methacrylic acid in a predetermined pattern to a formed fibrous substrate to produce a first composite comprising said fibrous substrate containing said solution arranged in said predetermined pattern; and

(b) irradiating said first composite with sufficient electromagnetic or corpuscular ionizing radiation to convert said salt of acrylic or methacrylic acid to a water-swellable polymer.

### The Prior Art

Assarsson et al., in U.S. Patent No. 3,901,236, disclose the production of absorbent articles by mixing an aqueous solution of various polymers, including a copolymer of sodium acrylate and acrylamide, with cellulosic fibers, followed by subjecting the resulting mixture to ionizing radiation.

It has been disclosed to employ radiation curable polymers as the bonding agents in producing nonwoven fabrics. For instance, in U.S. Patent No. 3,878,019 (Chapman et al.), nonwoven fabrics are produced by applying a film-forming polymer to a fibrous web substrate and subsequently cross-linking the polymer by the use of ultraviolet radiation. In U.S. Patent No. 4,091,140 (Harmon), a radiation curable polymer in the form of a continuous filament is employed as a bonding agent in a nonwoven fabric. Similar disclosures are found in U.S. Patent Nos. 4,146,417 (Drelich et al.), 3,709,738 (Wetherell), and 3,265,527 (Adelman).

Parker, in U.S. Patent No. 3,770,490, discloses the production of coatings by subjecting a solution of an acrylic polymer in acrylic monomer to ionizing radiation.

U.S. Patent No. 3,090,736 (Bashaw et al.) discloses the productin of insoluble, cross-linked products by subjecting aqueous solutions of salts of acrylic acid or polyacrylic acid to ionizing radiation.

Restaino, in U.S. Patent No. 3,764,502, discloses the production of polymers of sodium acrylate by irradiating aqueous solutions of sodium acrylate with high energy ionizing radiation. Phalangas, U.S. Patent No. 3,948,740, and Phalangas et al., U.S. Patent No. 4,024,040, disclose the production of water-soluble, substantially linear, high molecular weight polymers by irradiating an aqueous solution of an ethylenically unsaturated monomer and a chemical, free-radical initiator. Among the monomers disclosed are salts of acrylic and methacrylic acids.

### Detailed Description of the Invention

An aqeuous solution of a salt comprising fully or partially neutralized acrylic or methacrylic acid is employed in the invention. The salt employed is an ammonium salt or an alkali metal salt such as a sodium or potassium salt. The degree of neutralization employed can vary in particular cases, in view of several factors. For instance, at the preferred high solution concentrations, sodium acrylate may begin to precipitate when the degree of neutralization begins to exceed about 85 per cent. Therefore, it is preferred to employ sodium acrylate at about a 60 to 85 per cent degree of neutralization. The more soluble ammonium and potassium acrylates, or mixed salt acrylates, can be employed at higher degrees of neutralization.

Pure methacrylate polymers do not cross-link under radiation. Therefore, methacrylate salts are used only in a mixture with acrylic salts or with a water-soluble cross-linking monomer, as explained below.

In order to reduce drying requirements, it is preferred to employ the salt in as concentrated a solution as solubility permits. Thus, sodium acrylate is ordinarily employed in concentrations up to 40 to 45 weight per cent. The maximum concentration of other salts can easily be determined through routine experimentation.

The preferred aqueous solution for use in the invention comprises an aqueous solution of sodium acrylate.

It is permissible to include other materials in the aqueous solution. Such materials include polyfunctional, ethylenically unsaturated compounds such as methylene-bis-acrylamide, and polyethylene glycol diacrylates or dimethacrylates such as tetraethylene glycol diacrylate. These materials are employed as cross-linking agents. The polyfunctional monomer is used in small amounts, for instance, in amounts of less than one mol per cent, based on moles of acrylate salt(s).

A water-soluble polymer can be employed as a viscosity adjusting agent, for example, to improve the printing or spraying characteristics of the salt solution. Examples include polyvinyl pyrrolidone and hydroxyethyl cellulose. Colloidal silica, a thixotropic agent, can be employed for the same purpose.

Finely divided fillers may also be employed as extenders. Examples include talc, clay, diatomaceous earth and perlite.

Small amounts of polyvalent metal ions may be added to the salt to provide ionic cross-linking. Illustrations include calcium, magnesium, and aluminum.

The aqueous solution is applied in a predetermined pattern onto a formed fibrous substrate. The fibrous substrate can be a loosely formed batt of fibers, a carded or an air-layed web, tissue paper, a woven fabric such as cotton gauze, a knitted fabric, or a nonwoven fabric. By "formed" fibrous substrate is meant that the fibrous substrate need not undergo any further web-forming operation in order to be employed in an article, although it may require cutting, bonding, shaping, etc., in order to be fabricated into an article. It is generally preferred to employ absorbent fibers in the fibrous substrate such as cellulosic fibers including wood pulp, rayon, and cotton. It is permissible, however, to include other types of fibers in the formed fibrous substrate.

The aquous solution is applied to the formed fibrous substrate in a predetermined pattern. It is preferred to employ an intermittent pattern such as an intermittent pattern of fine dots or intermittent stripes. The pattern can be employed to produce "dams" or "wicking channels", in the absorbent composite that is produced by the process of the invention. For instance, a diaper having a continuous stripe of cross-linked absorbent polymer around the edges of the absorbent padding portion of the diaper will have less tendency to leak around the edges. In general, it is preferred to employ a pattern of very finely divided discrete areas in order to provide as high a ratio of polymer surface area to mass as possible. The reason for this is to utilize the absorbent capacity of the polymer to the fullest extent possible.

The aqueous solution can be applied to the fibrous substrate in the predetermined pattern by means such as printing, spraying, flowing through nozzles, kiss coating or saturating.

If desired, the aqueous solution can be applied to the fibrous substrate in an overall pattern, which may be applied in an amount sufficient to simply coat one surface of the fibrous substrate or it can be employed in a quantity sufficient to penetrate as much of the thickness of the fibrous substrate as is desired in particular cases.

The amount of aqueous solution added to the fibrous substrate is not narrowly critical, and can vary over a rather wide range, depending on factors such as end-use application for the product, and similar considerations. Thus, the add-on (on a solids basis) can vary from less than one per cent up to one hundred per cent, based on weight of fibrous substrate.

After the aqueous solution has been applied to the fibrous substrate, the material is then irradiated by electromagnetic or corpuscular ionizing radiation such as accelerated electrons or gamma rays sufficient to convert the acrylic and/or methacrylic salt to a water-swellable polymer. The dose employed in particular cases will vary somewhat, depending on factors such as presence or absence of cross-linking monomers, desired degree of polymerization of the polymer and degree of cross-linking desired. In general, it is desired to irradiate the first composite with doses in excess of two megarads, and preferably in excess of three megarads. Particularly when using lower doses, it may be desirable to purge oxygen from the aqueous salt solution (as by bubbling nitrogen through the solution). The maximum dose would be that dose at which degradation of the substrate begins. With cellulosic substrates, the literature reports that the dose at which degradation begins is about six megarads when gamma radiation is employed. Other forms of radiation would be expected to cause degradation at about the same dose.

After irradiating, the fibrous substrate may be dried to remove water by means such as by passing the composite over a series of drying cans, by the use of forced air ovens or by infrared lamps.

The following examples illustrate the practice of the invention:

### Example 1

An aqueous solution of 85 per cent neutralized sodium acrylate is prepared by mixing 50 per cent aqueous acrylic acid solution with 50 per cent aqueous sodium hydroxide solution to a pH of about 6. The resulting solution contains about 43 per cent solids. Nitrogen gas is bubbled through the solution to purge it of oxygen.

The foregoing solution is applied to a double layer web of woven cotton gauze. The web of gauze is passed under a series of drip nozzles from which the sodium acrylate solution flows under moderate pressure. The add-on, on a solids basis, is about 50 percent. The gauze is carried under the flow nozzles on a continuous stainless steel belt. The gauze containing continuous stripes of sodium acrylate solution is carried from the flow nozzles by said belt to a station where it is irradiated with accelerated electrons. The electron beam apparatus is a Dynamitron accelerator capable of providing a voltage of 800 kv. The gauge having the stripes of sodium acrylate solution thereon is irradiated with a dose of 4 megarads from this electron beam accelerator. After passing under the electron beam apparatus, the web is then passed over a series of drying cans in order to dry the water from the impregnated material. The dried product is a gauze material having continuous stripes of water-swellable polymer impregnated therein. It is useful as the absorbent layer of incontinence pads, diapers, surgical bandages, and similar articles.

### Example 2

In this example, in which the procedure is analogous to that described in Example 1, a web of soft and lofty, through-bonded nonwoven fabric weighing about 0.27 kg/m² (8 ounces per square yard) is employed. The fabric contains about 25 weight per cent rayon staple fibers and about 75 per cent wood pulp fibers. (The fabric is more particularly described by Liloia et al. in U.S. Patent No. 3,663,238.) The sodium acrylate solution described in Example 1 is applied sparingly to one surface of a continuous web of said fabric in a fine spray. The add-on level (solids basis) is about 20 per cent, by weight. As in Example 1, the web is then passes under the Dynamitron electron beam accelerator and is irradiated with a dose of 4 megarads. The web is then passed over a set of drying cans.

The dried product is an absorbent pad having one surface impregnated to a shallow depth with a water-swellable polymer. The product is especially useful in fabricating panty shields or similar sanitary protection articles. The product would be designed so that the impregnated surface would be worn away from the body.

### Example 3

By a procedure analogous to that described in Example 1, a continuous web of a print-bonded, non-apertured, rayon nonwoven fabric weighing 775 g/m² (600 grains per square yard), and made in accordance with the general teachings of U.S. Patent Nos. 3,705,687 and 2,705,688, is printed with the sodium acrylate solution of Example 1. The printing is done with an engraved print roll by a process analogous to that described by Drelich in U.S. Patent No. 4,084,033. The print roll contains a pattern of spaced, fine, round depressions about 5.1 mm (0.02 inch deep) and about 12.7 mm (0.05 inch) in diameter, 2.6 (six) depressions to the centimeter (inch in each direction in an alternating pattern. The add-on (solids basis) is about 30 per cent. The printed web is irradiated with a dose of 4 megarads by the Dynamitron electron beam accelerator, and is then dried. The product is an absorbent fabric useful as the absorbent layer in surgical bandages and similar products.

### Example 4

By a procedure analogous to that described in Example 1, a continuous 25.4 cm (10-inch) wide web of loosely compacted short cellulose fibers weighing about 12.92 g/m² (1000 grains per square yard), such as is described by Mesek et al. in U.S. Patent No. 3,612,055, is passed under a station wherein two continuous stripes of the aqueous sodium acrylate solution are applied to the web by flow nozzles. The add-on (solids basis) is about 30 per cent. The stripes are applied adjacent to both edges of the web. The web is then passed under the Dynamitron electron beam accelerator where it is irradiated with a dose of 4 megarads. After drying, the web product is especially useful as the absorbent pad in a disposable diaper, wherein the stripes of water-swellable polymer at the edges act as dams to retard leakage around the edges.

### Example 5

Example 1 is repeated except that 0.02 mol per cent of tetraethylene glycol diacrylate is added to the aqueous solution.

The product is an absorbent fabric useful as the absorbent layer in incontinence pads and similar articles.

### Example 6

Example 1 is repeated except that the acid used is a 50/50 (mol/mol) mixture of acrylic and methacrylic acid, and the dosage is increased to about 5-1/2 megarads. The absorbent product is useful as the absorbent layer in incontinence pads and similar articles.

## Example 7

Example 1 is repeated except that 10 mol per cent of the sodium hydroxide is replaced with calcium hydroxide. The absorbent product is useful as the absorbent layer in incontinence pads and similar articles.

## Claims

1. Process for producing an absorbent composite which comprises:

(a) applying an aqueous solution comprising an ammonium or alkali metal salt of acrylic or methacrylic acid in a predetermined intermittent pattern to a formed fibrous substrate to produce a first composite comprising said fibrous substrate containing said aqueous solution arranged in said predetermined pattern; and

(b) irradiating said first composite with sufficient electromagnetic or corpuscular ionizing radiation to convert said salt of acrylic or methacrylic acid to a water-swellable polymer.

2. The process of claim 1 wherein said salt is an alkali metal salt.

3. The process of claim 2 wherein the alkali metal is sodium.

4. The process of any one of claims 1 to 3 wherein the acid is acrylic acid.

5. The process of any one of claims 1 to 4 wherein the aqueous solution contains a small amount of water-soluble, polyfunctional, ethylenically unsaturated compound.

6. The process of claim 5 wherein said compound is methylene-bis-acrylamide or a polyethylene glycol diacrylate or dimethacrylate.

7. The process of any one of claims 1 to 6 wherein the aqueous solution contains a small amount of a water-soluble polymer.

8. The process of any one of Claims 1 to 7 wherein the fibrous substrate includes cellulosic fibers.

9. The process of claim 8 wherein the fibrous substrate is a loose batt of fibers, a carded web, an air-layed web, a paper, a nonwoven fabric, a woven fabric, or a knitted fabric.

10. The process of any of Claims 1 to 9 wherein the electromagnetic or corpuscular ionizing radiation is accelerated electrons.

11. The process of any of claims 1 to 10 which includes the additional step of drying the product of step (b).

## Revendications

1. Procédé de production d'un composite absorbant caractérisé en ce qu'il comprend:

(a) l'application d'une solution aqueuse comprenant un sel d'ammonium ou de métal alcalin de l'acide acrylique ou méthacrylique suivant un motif intermittent prédéterminé à un substrat fibreux formé pour produire un premier composite comprenant ledit substrat fibreux contenant ladite solution aqueuse disposée suivant ledit motif prédéterminé; et

(b) l'irradiation dudit premier composite avec une radiation ionisante électromagnétique ou corpusculaire suffisante pour convertir ledit sel de l'acide acrylique ou méthacrylique en un polymère gonflable dans l'eau.

2. Procédé selon la revendication 1, caractérisé en ce que ledit sel est un sel de métal alcalin.

3. Procédé selon la revendication 2, caractérisé en que le métal alcalin est du sodium.

4. Procédé selon l'une des revendications 1 à 3, caractérisé en ce que l'acide est l'acide acrylique.

5. Procédé selon l'une des revendications 1 à 4, caractérisé en ce que la solution aqueuse contient une petite quantité de composé insaturé éthyléniquement, polyfonctionnel, soluble dans l'eau.

6. Procédé selon la revendication 5, caractérisé en ce que ledit composé est le méthylène-bisacrylamide ou un diacrylate ou un diméthacrylate de polyéthylène glycol.

7. Procédé selon l'une des revendications 1 à 6, caractérisé en ce que la solution aqueuse contient une petite quantité d'un polymère soluble dans l'eau.

8. Procédé selon l'une des revendications 1 à 7, caractérisé en ce que le substrat fibreux comprend des fibres cellulosiques.

9. Procédé selon la revendication 8, caractérisé en ce que le substrat fibreux est un assemblage lâche de fibres, un tissu cardé, un tissu à couche d'air, un papier, un tissu non tissé, un tissu tissé, ou un tissu tricoté.

10. Procédé selon l'une revendications 1 à 9, caractérisé en ce que la radiation ionisante électromagnétique ou corpusculaire est constituée d'électrons accélérés.

11. Procédé selon l'une des revendications 1 à 10, caractérisé en ce qu'il comporte l'étape additionnelle de séchage du produit de l'étape (b).

## Patentansprüche

1. Verfahren zum Herstellen eines zusammengesetzten, absorbierenden Materials, dadurch gekennzeichnet, daß man

a) eine ein Ammonium- oder Alkalimetallsalz der Acrylsäure oder Methacrylsäure enthaltende wäßrige Lösung in einem vorbestimmten unterbrochenen Muster auf ein geformtes Fasersubstrat aufbringt zur Erzeugung eines ersten, aus diesem Fasersubstrat bestehenden zusammengesetzten Materials, das die wäßrige Lösung in dem vorbestimmten Muster angeordnet enthält, und

b) dieses erste zusammengesetzte Material zur Umwandlung des Acrylsäure- oder Methacrylsäuresalzes in ein in Wasser quellbares Polymer in ausreichendem Maße mit elektromagnetischen oder ionisierenden Korpuskularstrahlen bestrahlt.

2. Verfahren nach Anspruch 1, dadurch gekennzeichnet, daß das Salz ein Alkalimetallsalz ist.

3. Verfahren nach Anspruch 2, dadurch gekennzeichnet, daß das Alkalimetall Natrium ist.

4. Verfahren nach einem der Ansprüche 1 bis 3, dadurch gekennzeichnet, daß die Säure Acrylsäure ist.

5. Verfahren nach einem der Ansprüche 1 bis 4, dadurch gekennzeichnet, daß die wäßrige Lösung eine kleine Menge einer wasserlöslichen, polyfunktionellen, äthylenisch ungesättigten Verbindung enthält.

6. Verfahren nach Anspruch 5, dadurch gekennzeichnet, daß die Verbindung Methylen-bis-acrylamid oder ein Polyäthylenglykoldiacrylat oder -dimethacrylat ist.

7. Verfahren nach einem der Ansprüche 1 bis 6, dadurch gekennzeichnet, daß die wäßrige Lösung eine kleine Menge eines wasserlöslichen Polymeren enthält.

8. Verfahren nach einem der Ansprüche 1 bis 7, dadurch gekennzeichnet, daß das Fasersubstrat Cellulosefasern enthält.

9. Verfahren nach Anspruch 8, dadurch gekennzeichnet, daß das Fasersubstrat eine lockere Faserwatte, eine kardierte Bahn, eine luftgelegte Bahn, ein Papier, ein ungewebter Faserstoff, ein Gewebe oder eine Wirkware ist.

10. Verfahren nach einem der Ansprüche 1 bis 9, dadurch gekennzeichnet, daß die elektromagnetische oder ionisierende Korpuskularstrahlung beschleunigte Elektronenstrahlen sind.

11. Verfahren nach einem der Ansprüche 1 bis 10, dadurch gekennzeichnet, daß das Produkt der Stufe b) zusätzlich getrocknet wird.